# EUROPEAN PATENT SPECIFICATION

(11) **EP 1 009 453 B1**
(45) Date of publication and mention of the grant of the patent: **29.11.2006**
(21) Application number: 97935342.2
(22) Date of filing: 08.08.1997
(51) Int. Cl.: A61M 5/00

(54) **INCREMENTALLY ADJUSTABLE DOSE SYRINGE**
STUFENWEISE EINSTELLBARE DOSIERUNGSSPRITZE
SERINGUE A REGLAGE DE DOSE INCREMENTIEL

(43) Date of publication of application: 21.06.2000
(73) Proprietor: Genesis Industries Incorporated, Spring Valley, Wisconsin 54767 (US)
(72) Inventor: ANDERSON, Mark, L., Spring Valley, WI 54767 (US)
(74) Representative: Bailey, Richard Alan
(86) International application number: PCT/US1997/014135
(87) International publication number: WO 1999/007421

(56) References cited:
- EP-A- 0 064 858
- FR-A- 2 612 782
- US-A- 3 563 240
- US-A- 4 153 056
- US-A- 4 246 898
- US-A- 4 275 729
- US-A- 4 654 035
- US-A- 4 874 385
- US-A- 5 344 409
- US-A- 5 385 558

## Description

### BACKGROUND OF THE INVENTION

### 1. Field of the Invention.

The present invention relates, generally, to apparatus and methods for delivering fluids. More particularly, the invention relates to a syringe having a plunger and barrel with a ring engaged on the plunger for limiting the motion of the plunger. The invention meters the amount of material dispensed from the barrel. The invention is optimally used to easily, repeatably and accurately deliver an incrementally adjustable dose of material. However, the invention also may have utility in other applications.

### 2. Background Information.

A prior art syringe is described in U.S. Pat. No. 4,153,056 issued May 8, 1979. A related patent is U.S. Pat. No. 3,563,240, issued February 17, 1971. The prior art syringe includes a cylinder, a plunger, and a measuring ring. The cylinder has a chamber, a cannula with a core at a first end, and an open second end. The cylinder also has exterior finger flanges disposed adjacent the open second end. The plunger has a piston head with a ring seal disposed in the cylinder for sealing the chamber at a first end of the plunger, a stem with four ribs in a cruciform shape fixedly connected to the piston head, and a thumb engaging flange fixedly connected to the four ribs at a second end of the plunger. Each of the ribs has an outer edge with a set of spaced notches. The notches of the ribs form an exterior thread. The measuring ring has an interior thread that is in mesh with the exterior thread. The ring measures and sets a selective dose volume of fluid in the chamber and bore. A problem with the prior art syringe is that it can be difficult to accurately set the measuring ring for a desired dose volume.

A syringe latch invented by Ennis, III et al. and assigned to applicant's assignee is described in U.S. Patent No. 5,344,409. The disclosed syringe latch controls or meters the amount of material or fluid expelled from a syringe. The syringe has a syringe body having a chamber, a discharge end portion, and an opposite open end portion. A plunger is received in the syringe body. A measuring ring, having an inner thread in mesh with an outer thread on the plunger, limits the stroke of the plunger and thus the amount of material expelled from the syringe. The measuring ring turns about the plunger on the meshing threads and includes latch means or detent areas for setting and holding the measuring ring to a rib at a predetermined position along the plunger. Two equally-spaced detent areas latch to one rib of the plunger every half turn of the measuring ring. The detent areas and thread pitch are calibrated so that each half turn of the syringe corresponds to a predetermined volume interval within the chamber. For example, if the syringe is designed so that each half turn of the measuring ring corresponds to a volume of 2 cubic centimeters (cc), then the syringe accurately meters dose volumes in 2 cc intervals, *i.e.* dose volume intervals of 2 cc, 4 cc, and 6 cc for example. Therefore, an operator can accurately adjust the dose volume merely by counting the number of times a detent area latches to a rib rather than using other indicia on the barrel. However, the operator is not able to easily and accurately meter doses that may fall between these 2cc intervals, for example 1 cc, 3 cc, and 5 cc doses, which do not correspond to a detent area on the measuring ring.

Applicant's invention provides an adjustable dose device which overcome the limitations and shortcomings of the known art. Specifically, applicant's invention provides an improved syringe for easily, repeatably, and accurately metering doses of material. An operator measures a dose by counting the number of times the measuring ring latches to the plunger rather than visually aligning the measuring ring or using other indicia on the syringe. In addition, the parts of applicant's invention are easily substituted to create different dose intervals. Another advantage of the syringe is that, because the syringe is capable of metering out incremental doses, medicinal fluid or other material can be distributed within the syringe rather than in a separate vile.

According to the invention there is provided an adjustable dose syringe comprising:
(a) a hollow syringe body having a discharge end portion and an opposite open end portion;
(b) a plunger slidable in said syringe body through said open end portion, said plunger having a piston head located within said syringe body including a seal formed thereon between said plunger and the interior of said syringe body, said plunger including a stem provided with an external thread; and
(c) a threaded nut having an internal thread of a pre-determined pitch angle which mates with said external thread of said plunger, said threaded nut further having an inner circumference; and characterised in that the stem is provided with four metering ribs, each of said metering ribs having an outer edge and a set of notches at said outer edge, said set of notches on each of said metering ribs forming said external thread, each of said metering ribs further having a latchable minor thread diameter, said threaded nut having a single internally disposed detent area for latching said nut to said plunger outside of said syringe bod said detent area latching with said latchable minor thread diameter of each of said metering ribs, in use, whereby a syringe dose is incrementally adjustable without the necessity of changing the pitch angle.

An operator sets a desired dose volume of material to be expelled from a syringe using the measuring ring and its detent area. The plunger and measuring ring may be substituted with others to obtain a different thread pitch, a different metering rib configuration, and/or a different detent area configuration, all of which can change the amount of material expelled as governed by each revolution or partial revolution of the measuring ring. For example, a different thread pitch may result in a dose volume of 2 cc rather than 1 cc for each revolution of the measuring ring.

A principal object of the present invention is to provide a syringe which will meter predetermined dose volumes of material. Another object of the present invention is to provide an incrementally adjustable syringe capable of being set to deliver various dose volume. A further object of the present invention is to provide a device that is easily set to the desired dose interval for repeatably metering out a desired volume of material from a single draw within the syringe.

The features, benefits and objects of this invention will become clear to those skilled in the art by reference to the following description, claims and drawings.
Figure 1 is an elevational view of a syringe not according to the invention.
Figure 2 is a longitudinal sectional view of the syringe shown in Figure 1.
Figure 3 is an enlarged partial sectional view of an edge portion of the plunger piston and seal arrangement.
Figure 4 is a partial perspective view illustrating the threaded stem of the plunger.
Figure 5 is a sectional view as taken along Line 5-5 of Figure 1.
Figure 6 is a sectional view as taken along Line 6-6 of Figure 5.
Figure 7a is a sectional view of an alternative arrangement (not according to the invention) showing the ribs of the plunger and the detent areas of the measuring ring.
Figure 7b is a sectional view of the ribs of Figure 7a showing the metering ribs and uniform ribs in greater detail.
Figure 8a is a sectional view of a syringe according to an embodiment of the invention showing the ribs of the plunger and the detent areas of the measuring ring.
Figure 8b is a sectional view of the ribs of 8a showing the metering ribs in greater detail.
Figure 9a is a sectional view of another arrangement (not according to the invention) showing the ribs of the plunger and the detent areas of the measuring ring.
Figure 9b is a sectional view of the ribs of 9a showing the metering ribs and uniform ribs in greater detail.
Figure 10a is a sectional view of another arrangement (not according to the invention) showing the ribs of the plunger and the detent areas of the measuring ring.

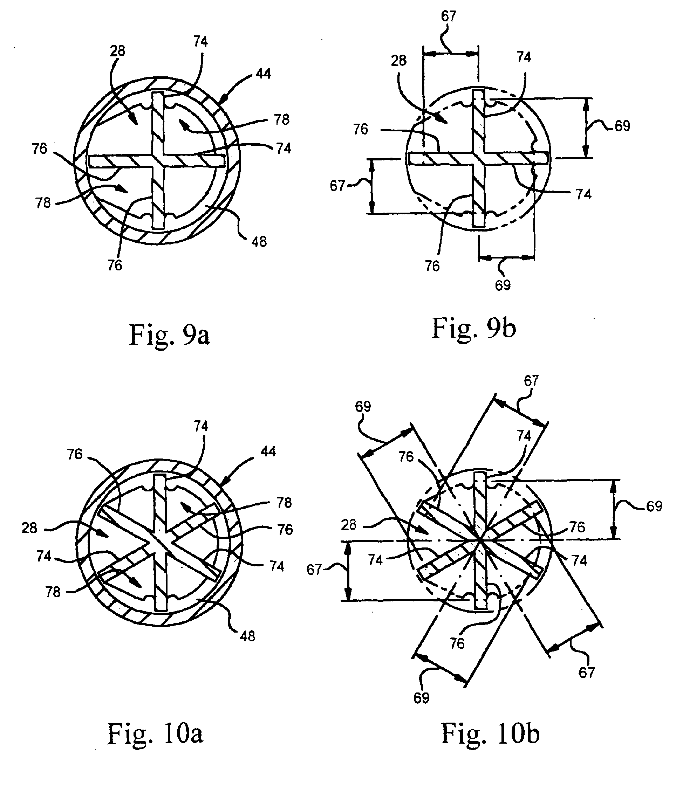

Figure 10 is a sectional view of the ribs of 10a showing the metering ribs and uniform ribs in greater detail.

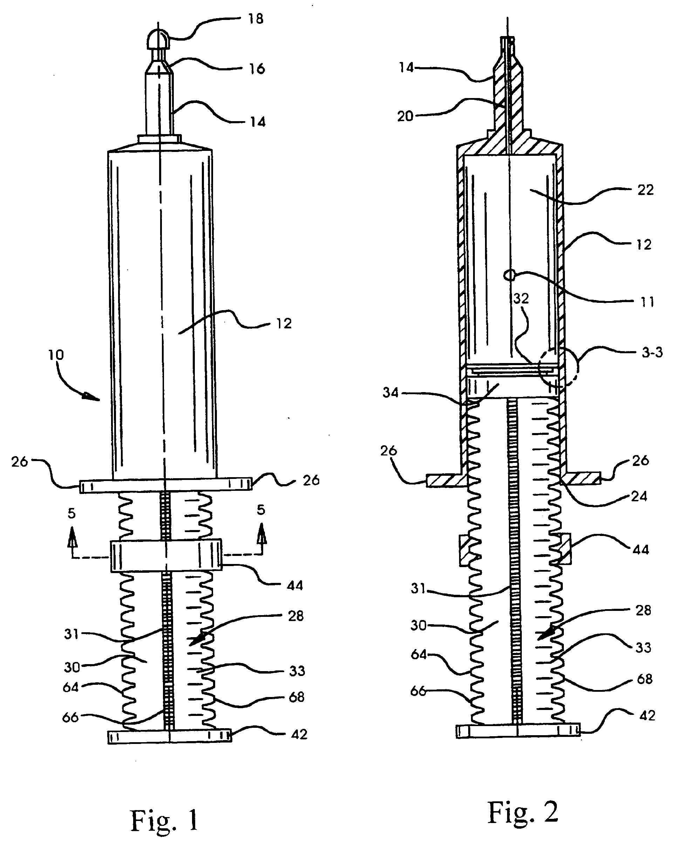

Figures 1 and 2 illustrate an incrementally adjustable dose syringe (not in accordance with the invention) generally designated by reference numeral 10 which has an axis 11 and includes a hollow syringe body or cylinder 12 formed of a synthetic plastic material, or the like, and includes an elongated tubular cannula 14 having a tapered end portion 16 that is effectively closed by a removable cap 18. The cannula 14 has a bore 20 formed therein which communicates with an internal chamber 22 of the syringe body 12. The chamber 22 is adapted to receive a pharmaceutical preparation or other material which is intended to be dispensed from the chamber 22 through the bore 20 of the cannula 14.

The chamber 22 has a uniform diameter from one end to the other and has an open end 24 opposite the cannula 14. Laterally extending handles or fmger flanges 26 are provided adjacent this end of the syringe body 12 in a conventional manner.

The material is expelled from the chamber 22 through the bore 20 by a plunger 28 having a stem portion or an integral body formed ofa plurality of radially extending ribs, 29, 30, 32, 33. The plunger 28 has a first or inner end portion 32 which includes an integral or snap on seal piston 34 formed thereon and adapted to be inserted through an open end 24 of the syringe body 12. The opposite end of the plunger 28 has an integral palm or thumb engaging base or flange 42 formed thereon in a conventional manner.

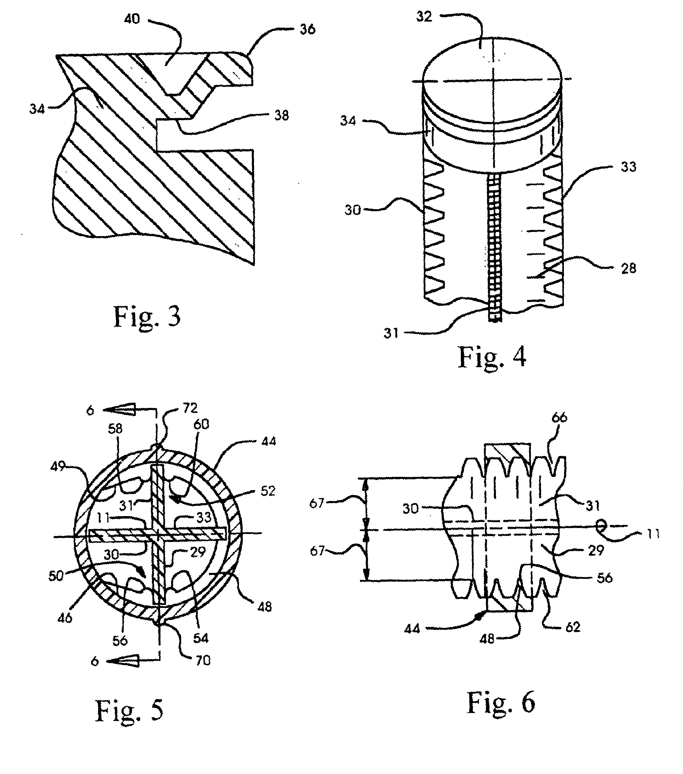

As shown in Figures 3 and 4, the seal piston 34 includes an integral annular seal or flange 36 surrounding the piston 34. The flange 36 has a diameter which is greater than the maximum diameter of the ribs 29, 30, 31, 33 of the plunger 28. The flange 36 is connected to the piston 34 by an integral annular web 38 which is formed in the piston 34 by an annular a V-shaped notch 40. The web 38 has a reduced thickness as compared to the flange 36 and is flexible to permit the flange 36 to serve as a wiping seal along the interior surface of the syringe body 12.

Referring again to Figures 1 and 2, a measuring ring or threaded nut 44 is provided on the plunger 28. The measuring ring 44 may be rotated to be properly located along the length of the plunger 28 to expel or meter a desired dose of material through the cannula 14. The measuring ring 44 generally has at least one detent area 78 and the plunger 28 generally has at least one metering rib 74 upon which the detent area 78 can be latched. An external thread is formed by notches in the metering rib or ribs 74 and any additional uniform ribs 76. The stroke of the plunger 28 and thus the volume of the expelled material is limited by adjusting the distance of the measuring ring 44 from the finger flanges 26. Furthermore, the measuring ring 44 can be repeatably set at incremental distances along the plunger 28 to dispense multiple doses from a single draw of material within the syringe.

As shown in Figure 5, the ring 44 has an inner surface 46 which has a segmental rib or internal thread 48 that has an inner face 49. In the embodiment shown, the thread 48 has as first detent area 50 and a second detent area 52, which are located 180 degrees apart. Each detent area is shown to have a first and second projection 54, 56 and 58, 60, each being made of a resilient conventional plastic material. Each projection extends from the inner circumference or inner face 49 in a radially inward direction. The device is shown with two detent areas 180 degrees apart, which causes the measuring ring 44 to latch with the metering rib 29 each time the ring 44 turns 180 degrees. The projections 54, 56 and 58, 60 respectively are disposed on the peripherally opposite sides of the ribs 29 and 31 in the latched condition.

As illustrated in Figure 2 and Figure 6, the ribs 29, 30, 31, 33 have along their outer edges respective sets of axially spaced notches 62, 64, 66, 68 that form an external thread which receive and mate with the internal thread 48 of the measuring ring 44. The three sets of notches 64, 66, 68 of the respective uniform ribs 30, 31, 33 have a similar minor thread diameter and radius 67. The fourth set of notches 62 found on the metering rib 29 has a minor thread diameter and radius 69 which is slightly larger than the uniform minor thread diameter and its radius 67. Thus, the latch 50 or 52 can only engage the plunger 28 on the fourth set of notches 62 of the metering rib 29. It should be noted that the minor thread diameter and radius extends to the valley or root of the notch and in a typical example, the axial width of each of the notches 62, 64, 66, 68 is slightly larger than the corresponding axial width of each of the projections 54, 56, 58, 60 and also larger than the corresponding axial width of the internal thread 48. Thus, the tip portions of the projections 54, 56 or 58, 60 of the detent areas 50 and 52 act to latch or hold the sides of one notch in the set of notches 62 adjacent to the root of the notch. It is anticipated that other detent area configurations and material could be used to latch the measuring ring 44 to the plunger 28.

The diameter of the chamber 22, the pitch angle of the threads, the number and arrangement of metering ribs 74, and the number and arrangement of detent areas 78 co-operate to provide the desired dose interval. A dose interval is the change of volume in the cylinder between successive latched positions of the measuring ring 44. In the embodiments shown in Figures 1-6, the measuring ring 44 is adjustable relative to the ribs 29, 30, 31, 33 by an angular displacement or rotation of the measuring ring 44 around the plunger 28. In particular, a half turn of the measuring ring 44 may provide a two cubic centimeter (cc) incremental volume change or dose interval in the chamber 22, including the bore 20. Other dose intervals may be accommodated by changing the diameter of the chamber 22 or the pitch angle of the internal and external threads.

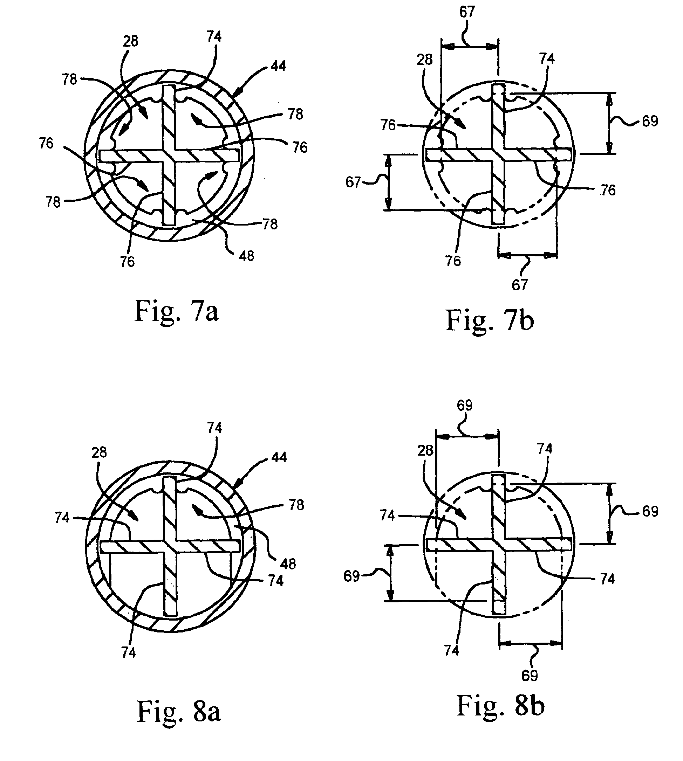

The measuring ring 44 and/or the plunger 28 are easily substituted to provide a different dose interval. Figures 7a and 7b (not according to the invention) show a measuring ring 44 having four detent areas 78 equally spaced 90 degrees apart from each other and a plunger 28 with one metering rib 74 and three uniform ribs 76. The measuring ring 44 latches onto the plunger 28 every quarter turn. This embodiment provides twice the number of dose intervals than the device that latches every half turn, and thus allows the device to meter out doses in relatively smaller dose increments. Alternatively, the plunger 28 may have multiple number of metering ribs 74. Figures 8a and 8b show a plunger 28 with four metering ribs 74 equally spaced 90 degrees apart and measuring ring 44 with one detent area 78. The measuring ring 44 still latches to the plunger 28 every quarter turn. Although it is preferable to use equally spaced detent areas and metering ribs to achieve repeatable incremental dose intervals, it is not required. Figures 9a and 9b (not according to the invention) show a plunger 28 with two metering ribs 74 at 0 degrees and 90 degrees respectively and a measuring ring 44 with two detent areas 78 and 90 degrees and 270 degrees respectively. The measuring ring 44 of this embodiment will also latch to the plunger 28 every quarter turn. Furthermore, the device can be configured to have multiple detent areas 78 and metering ribs 74. Figures 10a and 10b (not according to the invention) show a plunger 28 with three equally spaced metering ribs 74 and three equally spaced uniformed ribs 76, and a measuring ring 44 with two equally spaced detent areas 78. The measuring ring 44 of this embodiment will latch to the plunger 28 every sixth of a turn. As these figures show, there are numerous possibilities regarding the number and arrangement of both the detent areas and the metering ribs to produce various dose increments.

The measuring ring 44 shown in the embodiment of Figure 5 has an outer surface with a pair of ribs 70, 72. Ribs 70, 72 are respectively disposed radially outwardly of the latches 50, 52 as an aid for lining up the ring 44 with the contacting outwardly of the latches 50, 52 as an aid for lining up the ring 44 with the contacting flange 29 of the plunger 28. In an alternate embodiment (not shown), painted markings or raised arrows may be used in place of the ribs 70 72. In another alternate embodiment (not illustrated), the measuring ring 44 may have a rough finish or rippled finish in place of the smooth finish on its outer surface; or the measuring ring 44 may have a plurality of equally angularly spaced ribs on its outer surface in addition to the ribs 70, 72 which have a painted marking.

The descriptions above and the accompanying drawings should be interpreted in the illustrative and not the limited sense. While the invention has been disclosed in connection with the preferred embodiment or embodiments thereof, it should be understood that there may be other embodiments which fall within the scope of the invention as defined by the following claims. Where a claim is expressed as a means or step for performing a specified function it is intended that such claim be construed to cover the corresponding structure, material, or acts described in the specification and equivalents thereof, including both structural equivalents and equivalent structures.

## Claims

1. An adjustable dose syringe comprising:
(a) a hollow syringe body (12) having a discharge end portion and an opposite open end portion;
(b) a plunger (28) slidable in said syringe body through said open end portion, said plunger having a piston head (34) located within said syringe body including a seal formed thereon between said plunger and the interior of said syringe body, said plunger including a stem provided with an external thread; and
(c) a threaded nut (44) having an internal thread of a pre-determined pitch which mates with said external thread of said plunger, said threaded nut further having an inner circumference; and **characterised in that** the stem is provided with four metering ribs (74), each of said metering ribs having an outer edge and a set of notches at said outer edge, said set of notches on each of said metering ribs (74) forming said external thread, each of said metering ribs further having a latchable minor thread diameter, said threaded nut (44) having a single internally disposed detent area (78) for latching said nut (44) to said plunger outside of said syringe body, said detent area (78) latching with said latchable minor thread diameter of each of said metering ribs, in use, whereby a syringe dose is incrementally adjustable without the necessity of changing the pitch angle.

2. A syringe according to Claim 1, wherein said detent area (78) is provided with first and second projections.

## Patentansprüche

1. Einstellbare Dosierungsspritze, umfassend:
(a) einen hohlen Spritzenköper (12) mit einem Entladungs-Endabschnitt und einem gegenüberliegenden offenen Endabschnitt;
(b) einen Tauchkolben (28), der im Spritzenköper durch den offenen Endabschnitt verschiebbar ist, wobei der Tauchkolben einen Kolbenkopf (34) aufweist, der innerhalb des Spritzenkörpers angebracht ist, und eine Dichtung, die darauf zwischen dem Tauchkolben und dem Inneren des Spritzenköpers gebildet ist, einschließt, wobei der Tauchkolben einen Schaft einschließt, der mit einem Außengewinde versehen ist; und
(c) eine Gewindemutter (44) mit einem Innengewinde eines vorbestimmten Schrittes, die in das Außengewinde des Tauchkolbens eingreift, wobei die Gewindemutter weiter einen Innenumfang aufweist und **dadurch gekennzeichnet, dass** der Schaft mit vier Messrippen (74) versehen ist, wobei jede Messrippe eine Außenkante und einen Satz Kerben an der Außenkante aufweist, wobei der Satz Kerben auf jeder der Messrippen (74) das Außengewinde bildet, wobei jede der Messrippen weiter einen einklinkbaren kleineren Gewindedurchmesser aufweist, wobei die Gewindemutter (44) einen einzigen innen angebrachten Anschlagbereich (78) aufweist, um die Mutter in den Tauchkolben außerhalb des Spritzenköpers einzuklinken, wobei der Anschlagbereich (78) mit dem kleineren einklinkbaren Gewindedurchmesser jeder der Messrippen in Verwendung einklinkt, wobei eine Spritzendosis ohne die Notwendigkeit, den Schrittwinkel zu ändern, stufenweise einstellbar ist.

2. Spritze nach Anspruch 1, wobei der Anschlagbereich (78) mit ersten und zweiten Vorsprüngen versehen ist.

## Revendications

1. Seringue à dose réglable, comprenant:
(a) un corps de seringue creux (12) pourvu d'une partie d'extrémité de décharge et d'une partie d'extrémité ouverte opposée;
(b) un piston (28) coulissant dans ledit corps de seringue à travers ladite partie d'extrémité ouverte, ledit piston comprenant une tête de piston (34) située à l'intérieur dudit corps de seringue, incluant un joint d'étanchéité formé sur la tête entre ledit piston et l'intérieur dudit corps de seringue, ledit piston incluant une tige pourvue d'un filet extérieur; et
(c) un écrou fileté (44) pourvu d'un filet intérieur d'un pas prédéterminé qui s'adapte audit filet extérieur dudit piston, ledit écrou fileté ayant, en outre, une circonférence intérieure; et **caractérisée par le fait que** la tige est pourvue de quatre nervures de dosage (74), chacune desdites nervures de dosage ayant un bord extérieur et étant pourvue d'un ensemble d'encoches au niveau dudit bord extérieur, ledit ensemble d'encoches sur chacune desdites nervures de dosage (74) formant ledit filet extérieur, chacune desdites nervures de dosage ayant, en outre, un diamètre de filet verrouillable mineur, ledit écrou fileté (44) étant pourvu d'une unique zone de butée (78) située sur l'intérieur pour verrouiller ledit écrou (44) sur ledit piston à l'extérieur dudit corps de seringue, ladite zone de butée (78) se verrouillant avec ledit petit diamètre de filet verrouillable de chacune desdites nervures de dosage, lors de l'utilisation, une dose de la seringue pouvant être réglée de manière incrémentielle sans qu'il soit nécessaire de modifier l'angle de pas.

2. Seringue selon la revendication 1, dans laquelle ladite zone de butée (78) est pourvue d'une première et d'une seconde saillies.
